Europäisches Patentamt

European Patent Office

Office européen des brevets

(11) Publication number: **0 240 186**
**A2**

## (12) EUROPEAN PATENT APPLICATION

(21) Application number: **87302051.5**

(22) Date of filing: **10.03.87**

(51) Int. Cl.⁴: **F25J 3/00** , **C07C 9/04**

(30) Priority: **01.04.86 US 847071**

(43) Date of publication of application:
**07.10.87 Bulletin 87/41**

(84) Designated Contracting States:
**BE DE ES FR GB GR IT NL SE**

(71) Applicant: **MCDERMOTT INTERNATIONAL, INC.**
**1010 Common Street PO Box 60035**
**New Orleans Louisiana 70160(US)**

(72) Inventor: **Aghili, Hafez Kermani**
**402 Houghton Road**
**Katy Texas 77450(US)**

(74) Representative: **Purvis, William Michael Cameron et al**
**D. Young & Co. 10 Staple Inn**
**London WC1V 7RD(GB)**

(54) Process for separating hydrocarbon gas constituents.

(57) In a process for separating natural gas constituents, a portion (62) of the residue gas from a demethaniser column (32) is cooled, expanded, partially condensed and recycled (72) to the top of the column (32). This alters the chemical equilibrium in the column (32) causing the lighter methane to vapourise and separate from the heavier constituents such as ethane, propane etc. The lighter methane constituent rises and is removed from the top of the demethaniser column (32) while the heavier constituents fall and are removed (60) from the bottom of the column.

FIG.1

EP 0 240 186 A2

# PROCESS FOR SEPARATING HYDROCARBON GAS CONSTITUENTS

The invention pertains to a method of separating hydrocarbon gas constituents.

The history of separating gas constituents from natural gas is a relatively recent one. In the 1940's the first prototype natural gas cryogenic turboexpander was built and in the 50's this turboexpander concept was applied to air plants, hydrogen plants, and helium purification plants. However, it was not until the 1960's that the first commercial natural gas turboexpander plant started operation. As commercial demand for these separated gases increased, many other such plants came into existence each with better and improved designs for separating the lighter elements (methane, ethane and propane) from the heavier elements (butane, pentane and their iso-components) contained in natural gas.

Many patents exist pertaining to these improvements with some of the more relevant patents being those to Gulsby (US-A-4,464,190 and US-A-4,453,958), Horton (US-A-4,410,342 and US-A-3,398,543), and Campbell, et al (US-A-4,278,457 and US-A-4,171,964). While each of these patents are improvements over their predecessors, none of them address the commercial need for nearly 100% gas separation and recovery.

According to the present invention there is provided a process for separating the constituents of a natural gas stream characterised by the steps of:

(a) lowering the temperature of the gas stream;

(b) supplying the lower temperature gas stream to a high pressure separator, the high pressure separator separating the gas stream into a predominantly vapour stream and a predominantly fluid stream;

(c) lowering the pressure of the predominantly vapour stream;

(d) supplying the lower pressure vapour stream to an upper region of a demethaniser column;

(e) lowering the pressure of the predominantly fluid stream;

(f) supplying the lower pressure fluid stream to the demethaniser column at a level below that at which the vapour stream is supplied;

(g) removing cold vapour residue gas from an upper region of the demethaniser column, the vapour residue gas predominantly comprising methane and other residual light vapours;

(h) passing the vapour residue gas through at least one heat exchanger to raise the temperature of the vapour residue gas;

(i) compressing the vapour residue to a higher pressure;

(j) drawing off a portion of the higher pressure vapour residue;

(k) lowering the temperature of the drawn-off higher pressure vapour residue;

(l) lowering the pressure of the higher pressure residue to produce a predominantly liquid stream;

(m) supplying the lower temperature predominantly liquid stream as reflux to the top of the demethaniser column whereby the addition of the reflux alters the chemical equilibrium existing in the top of the demethaniser column thereby enhancing the separation of the inlet stream constitutents in the demethaniser column; and

(n) removing a demethanised liquid product from a lower region of the demethaniser column.

Such a process can achieve 100% separation of the natural gas feed stream constituents and can utilise a true distillation column for the separation of these constituents. 100% gas constitutent separation can be achieved in existing plants without requiring major plant restructuring.

Thus light weight methane can be separated from the heavier constituents found in a natural gas stream using a demethanising tower having one or more feed lines, one or more reboiler lines (or heat exchange lines) and a true reflux system. Preferably a high pressure separator and a gas expander are employed for the incoming feed line. A residue gas line coming from the top of the feed demethaniser column is used to chill the incoming natural gas before the residue gas is compressed and air cooled. A portion of this residue gas is subsequently chilled and then expanded in the reflux expander or by a flash control valve and returned to the top of the demethaniser column. This return line enables the demethaniser to operate as a true distillation column thereby achieving nearly 100% separation of ethane and heavier constituents from the lighter methane.

The invention is diagrammatically illustrated by way of example in the accompanying drawings, in which:-

Figure 1 is a schematic illustration of apparatus for use in carrying out a process according to the invention and showing how the various components correlate, including a demethaniser having a return reflux line;

Figure 2 is an alternate schematic illustration of apparatus for carrying out a process according to the invention wherein residue gas from a demethaniser flows first through a reflux exchanger before passing through a heat exchanger; and

Figure 3 is a further schematic illustration of apparatus for carrying out a process according to the invention wherein a reflux line is chilled via multiple heat exchangers.

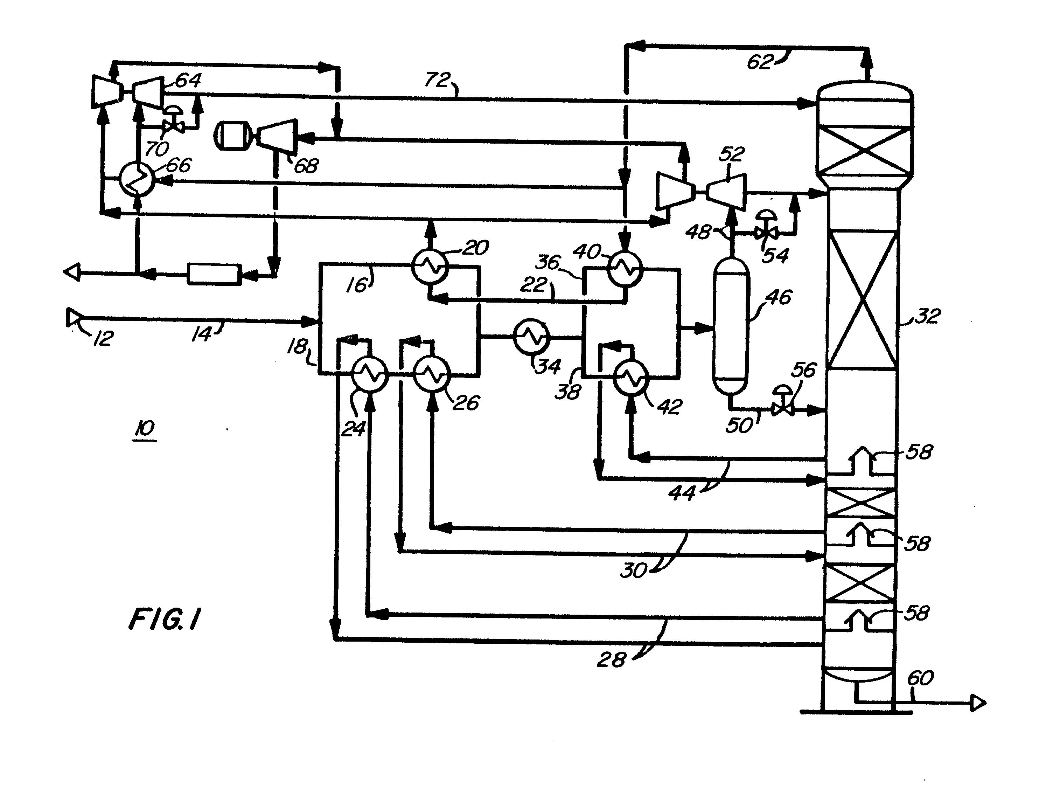

Referring to Figure 1, natural gas enters gas separator process apparatus 10 through an inlet 12 after having first been dehydrated. The gas flows in a line 14 and is divided into separate gas streams 16 and 18 respectively. The gas stream 16 is cooled in a warm gas/gas exchanger 20 by residue gas in a line 22. The gas stream 18 is cooled in a reboiler 24 and a lower side heater 26 through which demethaniser liquid flows via lines 28 and 30 from a demethaniser column 32.

From these exchangers, the cooled gas streams 16 and 18 recombine and enter a gas chiller 34 where the combined stream is further cooled by a refrigerant. After the chiller 34, the chilled stream is again separated into two portions, comprising a stream 36 and a stream 38, for more cooling. The stream 36 is cooled in a cold gas/gas exchanger 40 by cold residue gas directly from the top of the demethaniser column 32. This residue gas is generally at a temperature of -101°C (-150°F). As shown, this cold residue gas passes first through the cold gas/gas exchanger 40 before travelling through the warm gas/gas exchanger 20 via the line 22. The stream 38 is cooled in an upper side heater 42 by demethaniser liquid flowing through lines 44 from the demethaniser column 32. This demethanised liquid is generally at a temperature of -90°C (-130°F).

The cold gas streams 36 and 38 then recombine and enter a high pressure separator 46 where the cold inlet gas is separated into a gas stream 48 and a liquid stream 50. The gas stream 48, which by this time consists predominantly of the lighter methanes, ethanes and propanes, is expanded to reduce its pressure such as by a main expander 52 or across an expansion valve 54. The expansion further cools the gas before it is fed into an upper region of the demethaniser column 32. The condensed liquid stream 50 from the high pressure separator 46 is also expanded, thereby reducing its pressure, such as across an expansion valve 56, before entering the side of the demethaniser column 32. By this time the liquid stream 50 consists predominantly of the heavier butanes, pentanes and their iso-components.

As the liquid is fed to the demethaniser column 32, it flows downward under the force of gravity. During its journey, the liquid is engaged by rising vapours which strip the methane from the liquid as it passes through the demethaniser column 32. This stripping operation produces a demethanised end product which is removed from the bottom of the demethaniser column 32 via a line 60. The rising methane vapours are generated from heat obtained from the heat exchangers 24, 26 and 42 via the lines 28, 30 and 44. The demethaniser column is equipped with packing or trays 58 to effect the distillation process.

A residue line 62 leaves the top of demethaniser column 32 where its temperature is approximately -101°C (-150°F). A portion of the residue line, which is predominantly methane, is routed to the cold gas/gas exchanger 40 and the warm gas/gas exchanger 20 to cool the incoming gas streams 36 and 16. From these heat exchangers, the warmed residue gas is compressed by the compression side of the main expander 52, as shown, or by a reflux expander 64. The remainder of the residue from the residue line 62 passes through a reflux heat exchanger 66, where it is warmed before being compressed by the compression side of the reflux expander 64. After initial compression, this vapour is further compressed by a turbine recompressor 68 after which it is cooled and transported elsewhere.

A portion of the residue gas is cooled or condensed by the reflux heat exchanger 66 and then either expanded into liquid form across the reflux expander 64 or across a flash valve 70. The predominantly liquified reflux is then returned to the top of the demethaniser column 32 via a reflux stream 72 where its addition radically alters the chemical equilibrium in the column 32 causing the heavier elements to remain in a liquid state falling to the bottom while causing more of the lighter methane to vapourise and rise in the column 32. It is this recycling of the reflux to the column 32 that results in the column 32 acting as a distillation column thereby achieving a higher degree of gas separation and recovery of the heavier elements from the natural gas feed.

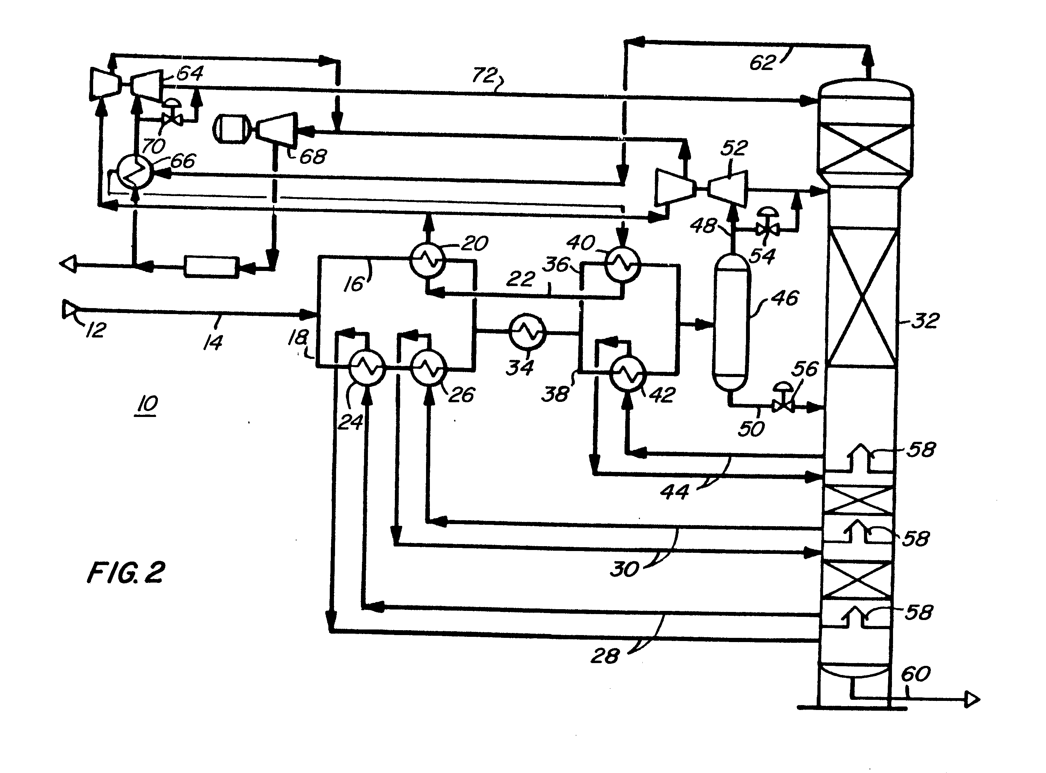

Another arrangement for the heat exchange of the reflux system is shown in Figure 2. In this case, the total residue stream coming from the demethaniser 32, via the residue line 62, flows through the reflux exchanger 66 first and then through the cold gas/gas exchanger 40 and the warm gas/gas exchanger 20 respectively.

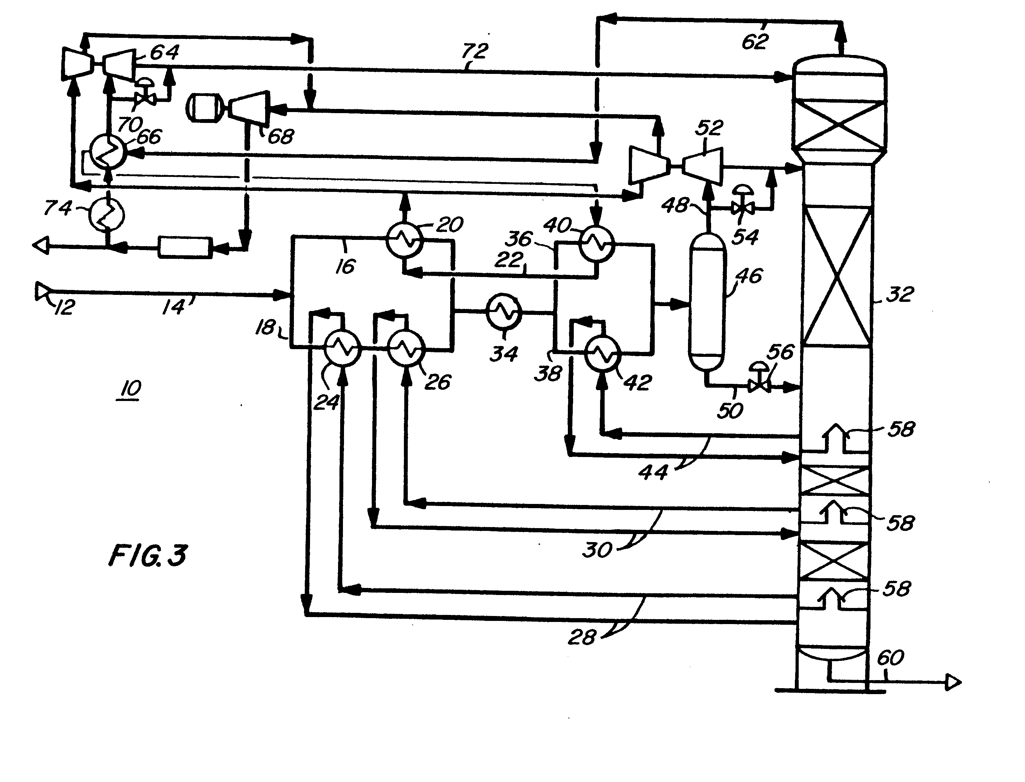

A further arrangement of apparatus for carrying out the gas separator process 10 is shown in Figure 3. In this case, the reflux stream 72 is first cooled by another medium such as cooling water or a refrigerant in a separate exchanger 74 before final cooling in the reflux exchanger 66.

Depending on the thermodynamic properties of the reflux stream 72 and the economics of the project, the reflux expander 64 may be used or the control valve 70 may be utilised to expand the portion of the residue gas returned to the demethaniser 32 via the reflux stream 72.

The inlet gas cooling arrangement comprising the exchangers 20, 24, 26, 34, 40 and 42 is a typical one. Other exchanger arrangements such as fewer side reboilers or a different refrigerant exchanger location may be utilised.

## Claims

1. A process for separating the constituents of a natural gas stream characterised by the steps of:

(a) lowering the temperature of the gas stream;

(b) supplying the lower temperature gas stream to a high pressure separator (46), the high pressure separator (46) separating the gas stream into a predominantly vapour stream (48) and a predominantly fluid stream (50);

(c) lowering the pressure of the predominantly vapour stream (48);

(d) supplying the lower pressure vapour stream to an upper region of a demethaniser column (32);

(e) lowering the pressure of the predominantly fluid stream (50);

(f) supplying the lower pressure fluid stream (50) to the demethaniser column (32) at a level below that at which the vapour stream (48) is supplied;

(g) removing cold vapour residue gas from an upper region of the demethaniser column (32), the vapour residue gas predominantly comprising methane and other residual light vapours;

(h) passing the vapour residue gas through at least one heat exchanger (66) to raise the temperature of the vapour residue gas;

(i) compressing the vapour residue to a higher pressure;

(j) drawing off a portion of the higher pressure vapour residue;

(k) lowering the temperature of the drawn-off higher pressure vapour residue;

(l) lowering the pressure of the higher pressure residue to produce a predominantly liquid stream;

(m) supplying the lower temperature predominantly liquid stream as reflux to the top of the demethaniser column (32) whereby the addition of the reflux alters the chemical equilibrium existing in the top of the demethaniser column thereby enhancing the separation of the inlet stream constituents in the demethaniser column; and

(n) removing a demethanised liquid product from a lower region of the demethaniser column.

2. A process according to claim 1, wherein the temperature of the gas stream is lowered by separating the stream into two streams (16, 18) and cooling the streams in gas heat exchangers (20, 24, 26).

3. A process according to claim 2, wherein the gas heat exchangers use the cold vapour residue gas as a refrigerant and the demethanised liquid product as a refrigerant.

4. A process according to claim 3, wherein the pressure of the predominantly vapour stream is lowered across expansion means.

5. A process according to claim 4, wherein the expansion means comprise a main expander (52).

6. A process according to claim 4, wherein the expansion means comprise an in-line expansion valve (54).

7. A process according to claim 4, wherein the pressure of the predominantly fluid stream (50) is lowered across an in-line expansion valve (56).

8. A process according to claim 7, wherein the cold vapour residue gas is compressed on the compressor side of an expander (52).

9. A process according to claim 8, wherein the temperature of the drawn-off vapour residue gas is lowered in a heat exchanger (66) using cold vapour residue gas as a refrigerant.

10. A process according to claim 8, wherein the pressure of the drawn-off vapour is lowered across expansion means.

11. A process according to claim 10, wherein the expansion means comprises a reflux expander (64).

12. A process according to claim 10, wherein the expansion means comprises an in-line flash valve (70).

13. A process according to claim 10, wherein the temperature of the gas stream is lowered in a chiller (74).

14. A process according to claim 13, wherein the demethaniser tower in a natural gas extraction plant is a distillation tower having a reboiler system and a reflux system.

FIG.1

0 240 186

FIG. 2

0 240 186

FIG. 3

0 240 186